# FASCICULE DE BREVET EUROPEEN

(11) **EP 0 594 769 B1**
(45) Date de publication et mention de la délivrance du brevet: **29.11.1995**
(21) Numéro de dépôt: 92916092.7
(22) Date de dépôt: 16.07.1992
(51) Int. Cl.: A61F 2/02, A61B 17/12

(54) **DISPOSITIF INTRAVEINEUX POUR L'APLATISSEMENT PARTIEL OU TOTAL D'UNE VEINE**
INTRAVENÖSE VORRICHTUNG ZUM VOLLSTÄNDIGEN ODER TEILWEISEN ABFLACHEN EINER VENE
INTRAVENOUS DEVICE FOR THE PARTIAL OR TOTAL FLATTENING OF A VEIN

(30) Priorité: 16.07.1991 FR 9108935
(43) Date de publication de la demande: 04.05.1994
(73) Titulaire: VAN CLEEF, Jean-Francois, F-75009 Paris (FR)
(72) Inventeur: VAN CLEEF, Jean-Francois, F-75009 Paris (FR)
(74) Mandataire: Hénnion, Jean-Claude
(86) Numéro de dépôt international: FR9200688
(87) Numéro de publication internationale: WO9301764

(56) Documents cités:
- DE-A- 3 203 410
- FR-A- 2 608 418
- US-A- 4 994 069

## Description

La présente invention concerne un dispositif intraveineux, relatif au domaine de l'angiologie et en particulier au traitement des varices, ledit dispositif permettant de réaliser un aplatissement partiel ou total de la lumière veineuse.

Traditionnellement les veines font l'objet de ligatures externes par fils ou clips. Certains médecins affirment que la pose de ressorts extraveineux éviterait la dilatation de ces veines et la formation des varices. D'autres envisagent de poser des valvules mécaniques, de type soupape, au niveau de la crosse de la saphène interne.

En ce qui concerne les dispositifs intraveineux, on connaît les dispositifs du type filtre pour la veine cave ou encore des endoprothèses cylindriques qui sont destinées à maintenir ou à renforcer l'ouverture de la lumière veineuse pour les greffes ou les shunts, à savoir les dérivations mettant en communication le circuit artériel et le circuit veineux.

On a déjà proposé dans le document FR.2.652.737 un filtre destiné à être mis en place dans la veine cave inférieure pour y arrêter des caillots susceptibles d'entraîner une embolie pulmonaire. Ce filtre est réalisé en un ressort rémanent conformé sous forme d'une spirale à trois spires non jointives dont la spire médiane a un plus grand diamètre que les deux autres. Ce filtre provoque un certain aplatissement de la veine cave dans laquelle il est mis en place ; mais du fait de sa forme en trois spires non jointives il n'est pas plan quand il est positionné dans la veine, ce qui empêche toute obstruction significative de la lumière veineuse, le flux sanguin étant simplement coupé en six segments semi-circulaires.

S'agissant de l'obstruction proprement dite de la lumière veineuse, on fait actuellement appel à des objets volumineux,tels que des ballonnets ou des cylindres , qui sont palpables sous la peau et qui engendrent des risques de migration et de thrombose très inflammatoire. Le but visé par le demandeur est de proposer un dispositif intraveineux qui pallie les inconvénients des dispositifs précités en ce qu'il permet de réaliser une obstruction partielle ou totale de la lumière veineuse sans risque de migration ou de thrombose.

Ce but est parfaitement atteint par le dispositif intraveineux selon l'invention. Il s'agit d'un dispositif pour aplatir partiellement ou totalement la lumière veineuse qui est sensiblement plan et qui comporte au moins deux tiges d'appui, lesdites tiges étant reliées par au moins un élément écarteur formant ressort et étant aptes à prendre appui respectivement sur l'un et l'autre des bords opposés d'une veine.

Le dispositif de l'invention se distingue nettement du dispositif d'obstruction, du document FR.2.608.408 , ce dernier ne possédant pas de tiges d'appui sensiblement parallèles lorsqu'il est en position d'utilisation et n'étant pas prévu pour écarter la veine mais pour l'obstruer par formation progressive d'un agrégat à partir du flux sanguin.

Pour concevoir son invention, le demandeur a mis à profit le constat qu'une veine , qui est un tube collabable présente un plan préférentiel d'aplatissement, qui est par exemple, dans le cas d'une veine superficielle, parallèle à la surface de la peau. Récemment, grâce à l'endoscopie veineuse, on a pu définir pour une veine , par exemple la veine saphène quatre éléments constitutifs à savoir une face interne ou profonde, une face externe ou superficielle et deux bords réunissant les deux faces. Dans la suite de la présente demande, l'appellation bord de la veine correspondra à l'un ou l'autre des deux éléments précités.

Au niveau d'une valvule, les bords de la veine sont matérialisés par les commissures, formées par la réunion des cornes valvulaires.

Le jeu valvulaire et la vitesse de circulation sanguine sont améliorés par l'ovalisation de la lumière veineuse.

Ainsi le dispositif intraveineux selon l'invention, lorsqu'il est positionné à l'intérieur de la veine, s'inscrit sensiblement dans un plan, qui dans le cas d'une veine superficielle est parallèle à la peau, c'est-à-dire qu'il respecte l'axe préférentiel d'aplatissement de la veine. Grâce à l'élément écarteur, il tend à éloigner les deux bords veineux l'un de l'autre et par conséquent à rapprocher les deux faces interne et externe de la veine.

On comprend que la surface d'appui, correspondant à la zone de contact entre les tiges d'appui et la paroi interne de la veine doit être suffisante pour éviter les risques de déchirures et de perforations , dus à la fragilité du tissu veineux.

On peut utiliser toutes sortes d'éléments écarteurs à effet ressort pour autant qu'ils puissent s'inscrire, le dispositif étant mis en place dans la veine, sensiblement dans un plan entre les tiges d'appui.

Selon un mode de réalisation, l'élément écarteur a une forme en X et il comporte quatre tiges d'appui disposées aux quatre extrémités dudit élément.

Selon un autre mode de réalisation, l'élément écarteur comporte des portions, de préférence aplaties, aptes à faire office de tiges d'appui. Bien sûr dans ce cas les portions susceptibles de faire office de tiges d'appui doivent être sensiblement rectilignes. Par exemple le dispositif a la forme d'une ellipse allongée et les deux tiges d'appui sont constituées par les portions en vis-à-vis , sensiblement parallèles au grand axe.

Dans un autre exemple l'élément écarteur consiste dans un fil ressort sinusoïdal dont les sommets aplatis forment les tiges d'appui.

Dans un autre exemple l'élément écarteur consiste dans une lame ressort en V, les extrémités libres des branches du V faisant office de tiges d'appui.

Avantageusement le dispositif intraveineux selon l'invention comporte des éléments d'ancrage, notamment situés sur tout ou partie des tiges d'appui. Ces éléments doivent permettre le blocage en position du dispositif par rapport à la paroi interne de la veine et empêcher toute migration de celui-ci.

Avantageusement les tiges d'appui sont légèrement ondulées, de manière à augmenter la surface de contact avec la paroi veineuse et par conséquent leur capacité d'ancrage.

Lorsque les tiges d'appui et l'élément écarteur sont indépendants les uns des autres, l'élément écarteur est fixé aux tiges d'appui soit par soudage soit à l'aide d'articulations, par exemple du type charnière.

Avantageusement le dispositif intraveineux de l'invention est équipé de moyens de réglage , d'une part permettant son adaptation au diamètre interne de la veine et d'autre part permettant de déterminer le degré d'aplatissement souhaitable de la veine. Grâce à ces moyens de réglage, il est possible d'utiliser un même dispositif pour réaliser soit un aplatissement partiel ou ovalisation de la veine soit un aplatissement total ou obstruction de la veine.

Selon un mode particulier de réalisation, l'élément écarteur a la forme d'un losange et les moyens de réglage consistent en un système de vis sans fin ou de crémaillère , placé selon l'axe longitudinal du losange.

Le dispositif intraveineux selon l'invention est sensiblement plan, lorsqu'il est positionné à l'intérieur de la veine. On comprend par là qu'il est parfaitement plan lorsque l'élément écarteur est strictement dans le même plan que les tiges d'appui. Cependant on considère qu'il est sensiblement plan lorsque tout ou partie de l'élément écarteur se trouve à une faible distance du plan formé par les tiges d'appui. En particulier ceci est obtenu dans les cas d'un dispositif ayant la forme d'un H avec au moins une barre transversale courbe, située au-dessus du plan formé par les tiges d'appui. De préférence le dispositif en H a deux barres transversales courbes, de courbures opposées, l'une étant située au-dessus et l'autre en-dessous du plan formé par les tiges d'appui. Dans ce cas les deux barres transversales délimitent une zone sensiblement ovale de passage du flux sanguin à l'intérieur de la veine. Cette forme correspond à un aplatissement partiel de la veine, les deux barres transversales s'opposant au rapprochement des deux faces interne et externe de la veine et donc à l'obstruction complète de celle-ci.

Ce mode particulier de réalisation est de préférence utilisé lorsque l'on veut combler l'espace intercornéal des valvules et renforcer la fonction d'arc tenseur du bourrelet valvulaire. Dans ce cas la courbure concave des barres transversales est déterminée de manière à épouser la forme des deux bourrelets valvulaires.

Avantageusement l'une au moins des tiges ou l'élément écarteur est dans un matériau apte à contenir et à diffuser des produits médicamenteux.

Avantageusement le dispositif intraveineux selon l'invention est réalisé, en tout ou partie , dans un matériau résorbable.

Selon un mode particulier de réalisation destiné à la section ou à la sclérose de la paroi veineuse, le dispositif intraveineux est composé de deux ensembles distincts , chaque ensemble étant constitué d'au moins deux tiges d'appui et d'un élément écarteur ; lesdits ensembles sont reliés par leurs éléments écarteurs à l'aide d'un lien résorbable ; les quatre tiges sont munies de moyens d'ancrage et le lien résorbable est imprégné d'un produit apte à réaliser la section ou la sclérose chimique de la paroi veineuse. Ainsi lorsque la diffusion du produit aura fait son effet, et que le lien aura été résorbé, les éléments écarteurs se trouveront respectivement de part et d'autre de la section de la veine et permettront l'obstruction complète des deux parties en vis-à-vis de celle-ci.

La présente invention sera mieux comprise à la lecture de la description qui va être faite de plusieurs modes de réalisation d'un dispositif intraveineux d'aplatissement partiel ou total d'une veine , comportant des tiges d'appui et un élément écarteur sensiblement dans un même plan , illustré par le dessin annexé dans lequel :
La figure 1 est une vue d'un dispositif en forme d'ellipse allongée.
La figure 2 est une vue d'un dispositif en forme de croix à deux tiges d'appui.
La figure 3 est une vue d'un dispositif en forme de H à une barre transversale dans le même plan que les tiges d'appui.
La figure 4 est une vue d'un dispositif en forme de H à deux barres transversales courbes.
La figure 5 est une vue d'un dispositif de forme sinusoïdale.
Les figures 6A et 6B sont deux vues d'un dispositif dont l'élément écarteur a la forme d'un V, à l'état libre (fig.6A) et placé dans la veine (fig 6B).
La figure 7 est une vue d'un dispositif en forme de X à quatre tiges.
La figure 8 est une vue d'un dispositif dont l'élément écarteur a la forme d'un losange muni d'un dispositif de réglage de l'écartement.
La figure 9 est une vue d'un dispositif à deux ensembles dissociables pour obstruction d'une veine.

Le dispositif intraveineux de l'invention est destiné à assurer l'aplatissement partiel, c'est-à-dire parfaire l'ovalisation d'une veine lorsque celle-ci est déficiente ou encore assurer l'aplatissement total, c'est-à-dire l'obstruction d'une veine.

On sait qu'une veine n'a pas une paroi circonférentiel lement homogène et présente une capacité d'aplatissement qui est préférentielle selon un plan déterminé, ce plan étant dans le cas d'une veine superficielle parallèle à la surface de la peau. Ainsi on a défini, pour une paroi veineuse, deux faces reliées par deux bords : la face interne ou profonde tournée vers l'intérieur du corps, la face externe ou superficielle tournée vers la peau et les deux bords qui réunissent ces deux faces.

Le dispositif selon l'invention doit pouvoir être utilisé pour remédier ou intervenir en cas de dysfonctionnement de la paroi veineuse.

Les moyens mis en oeuvre dans ce dispositif ont pour fonction d'éloigner dans une certaine mesure les deux bords veineux et par conséquent de rapprocher les deux faces interne et externe l'une de l'autre. Un tel rapprochement peut conduire soit à une meilleure ovalisation de la lumière veineuse soit éventuellement au rapprochement complet des deux faces veineuses et donc l'obstruction de la lumière veineuse.

Les moyens mis en oeuvre consistent dans au moins deux tiges d'appui 1 et 2 reliées par un élément écarteur 3.

Les deux tiges d'appui 1 et 2 sont destinées , lorsque le dispositif 4 est placé à l'intérieur de la veine , à venir s'appliquer respectivement le long de chacun des deux bords de la veine. L'élément écarteur, formant ressort, doit exercer une certaine force de pression sur chacune des deux tiges 1 et 2 de manière à les repousser l'une de l'autre et à les appliquer contre les deux bords veineux. Ainsi ces deux tiges 1 et 2 sont sensiblement parallèles lorsqu'elles sont disposées à l'intérieur de la veine.

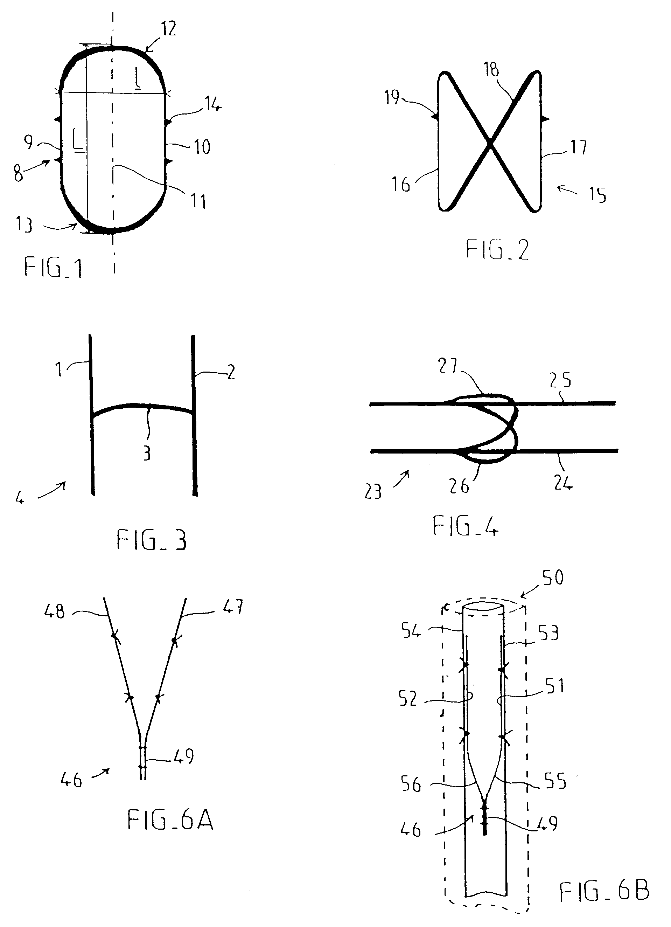

Le dispositif 4 représenté à la figure 3 est un exemple très simple de réalisation dans lequel l'élément écarteur 3 est un fil métallique . Ce dispositif 4 a la forme d'un H, l'élément écarteur 3 constituant la barre transversale et ayant une forme courbe dans le même plan que les deux tiges 1 et 2.

D'autres formes d'écarteurs sont envisageables par exemple en Ω , en fer à cheval , en N , en 7, en T , en croix de Lorraine, en échelle, en Y en Z, en S, en triangle, en ovale.

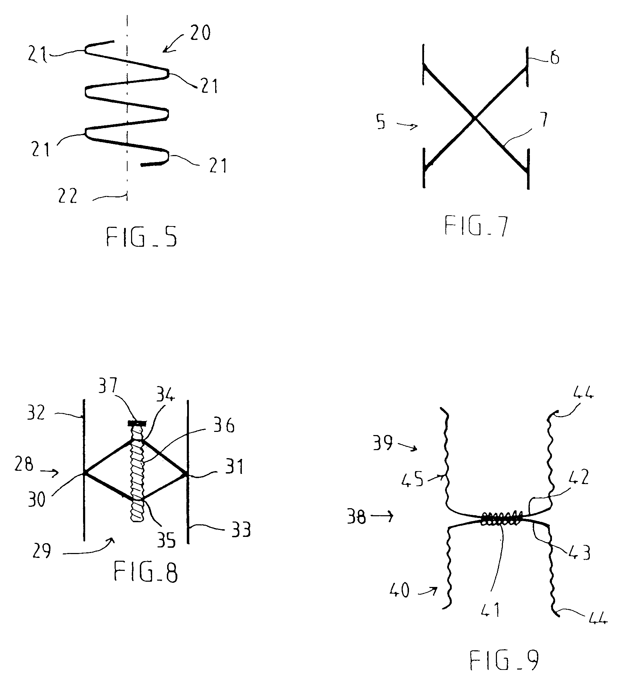

A la figure 7 on a représenté un dispositif 5 qui comporte quatre tiges d'appui 6, de plus petites dimensions qui sont fixées respectivement aux quatre extrémités de l'élément écarteur 7 en forme de X.

Dans tous les exemples qui viennent d'être décrits , les tiges d'appui sont dissociées de l'élément écarteur proprement dit. Dans ce cas la fixation des tiges sur l'élément écarteur est réalisée notamment par soudage ou à l'aide d'articulations, notamment du type charnière.

Afin de simplifier la réalisation matérielle du dispositif, il est bien sûr préférable que ce dispositif soit monobloc et ne comporte aucun point de fixation entre ces différents éléments. Les trois exemples montrés aux figures 1, 2 et 5 sont des réalisations de dispositifs monoblocs, dans lesquelles les tiges d'appui sont des portions de l'élément écarteur.

A la figure 1 on a représenté un dispositif 8 qui consiste en une ellipse métallique de forme allongée, ayant une longueur L, selon le grand axe 11 de l'ellipse. Cette ellipse est remarquable en ce qu'elle comporte, parallèlement à cet axe 11, deux portions sensiblement rectilignes 9,10, qui font office de tiges d'appui. Ce dispositif 8 comporte donc deux éléments écarteurs 12,13 consistant dans les extrémités courbes de l'ellipse, reliant les portions 9, 10.

Dans un mode précis de réalisation, ce dispositif 8 était réalisé à l'aide d'un fil chrome cobalt dans la proportion de 40/60 .Ce fil était de section circulaire de diamètre de 0,3mm au niveau des deux extrémités courbes 12, 13 et aplati sensiblement sous forme d'un ruban de 4mm de largeur au niveau des deux portions rectilignes 9,10 faisant office de tiges d'appui. En position intraveineuse, le dispositif 8 de la figure 1 avait une longueur L de 3cm de long et une largeur 1 de 8mm. De plus les portions 9,10 sont munies de dents 14, tournées vers l'extérieur de l'ellipse, de manière à assurer l'ancrage du dispositif 8 dans la paroi veineuse.

A la figure 2, on a représenté un dispositif 15 comportant deux tiges 16,17 reliées par un élément écarteur 18 en forme de X. Chacune des tiges 16,17 est en fait un prolongement rectiligne de deux extrémités latérales du X. Sur la face externe , tournée vers l'extérieur du X, de chaque tige 16,17 est prévue une pointe d'ancrage 19, placée sensiblement aux deux tiers de la longueur de ladite tige.

A la figure 5 on a représenté un dispositif 20 ayant la forme d'une sinusoïde. Cette sinusoïde est réalisée à l'aide d'un fil métallique. Les huit sommets 21, 4 de part et d'autre de l'axe transversal 22 de la sinusoïde , sont aplatis et constituent des tiges d'appui de dimension plus réduite.

Dans tous les exemples qui viennent d'être décrits, l'élément écarteur se trouve strictement dans le même plan que les tiges d'appui. Ces versions conviennent en particulier pour obtenir soit l'ovalisation soit l'obstruction de la lumière veineuse.

Dans l'exemple illustré à la figure 4, le dispositif 23 ne peut servir qu'à l'ovalisation de la lumière veineuse et non à son obstruction. Ce dispositif 23 a une forme de H, avec deux tiges d'appui 24,25 reliées par deux barres transversales 26,27. Ces deux barres transversales 26,27 sont courbes. L'une d'elles 27 se trouve, dans la représentation de la figure 4, située au-dessus du plan des tiges 24,25 tandis que l'autre 26 se trouve située sous ce plan. On comprend que lorsque le dispositif 23 est mis en place dans la veine, et que les tiges 24 et 25 sont appliquées sur les bords veineux, les deux barres transversales 26,27 formant l'élément écarteur délimitent une zone de passage pour le flux sanguin et empêchent les faces interne et externe de la paroi veineuse de venir en contact l'une de l'autre.

Cette version particulière, illustrée à la figure 4 est spécialement utile pour réaliser une consolidation valvulaire. Dans ce cas la courbure des barres transversales 26,27 est déterminée en sorte que celles-ci épousent, une fois l'extrémité des barres placée dans l'espace intercornéal, la forme des bourrelets valvulaires. Ainsi l'action des tiges d'appui, repoussées l'une de l'autre par les éléments écarteurs, tendent les bords libres de valvules et renforcent la fonction d'arc tenseur des bourrelets valvulaires.

Le dispositif 28 montré à la figure 8 comporte des moyens de réglage permettant son adaptation à la géométrie de la lumière veineuse ou bien de déterminer le degré d'aplatissement souhaité de la veine. Lesdits moyens de réglage consistent dans un élément écarteur 29 en forme de losange dont deux sommets 30,31 opposés sont fixés aux deux tiges d'appui 32,33 et dont les deux autres sommets 34,35 sont pourvus de trous taraudés. Une tige filetée 36, relie les deux sommets 34,35 et est équipée à une de ses extrémités d'une vis de réglage 37. Ainsi il est possible , à l'aide de la vis de réglage 37 de faire varier l'angle intérieur du losange et donc de moduler l'effort de tension impliqué vers les tiges 32,33 par le système écarteur 29.

L'exemple de réalisation illustré à la figure 9 concerne plus spécifiquement la section ou la sclérose d'une veine. Ce dispositif 38 est composé de deux ensembles 39,40 reliés à l'aide d'un lien 41. Ces deux ensembles 39,40 ont la forme d'un U et sont accolés par leur base 42,43 qui fait office d'élément écarteur tandis que les branches du U font office de tiges d'appui. Chacune de ces branches est terminée par un crochet 44 tourné vers l'extérieur et permettant l'ancrage du dispositif 38 lorsqu'il est mis en place dans la veine. Les deux bases 42,43 sont réunies par un fil 41 de catgut résorbable, imprégné de produit sclérosant.

Lorsque le dispositif 38 est mis en place dans la veine, la diffusion du produit sclérosant sectionne la veine tandis que les deux éléments 39,40 ancrés de part et d'autre de la zone sectionnée assurent le maintien en place des deux extrémités de la veine sectionnée. Bien sûr l'effort d'extension des bases 42 et 43 est déterminé en sorte de réaliser l'aplatissement total de la veine et donc l'obstruction de celle-ci au niveau des extrémités sectionnées.

Dans l'exemple de réalisation illustré à la figure 6A, le dispositif 46 a la forme d'un V. Il est constitué de deux lames 47,48 solidarisées l'une contre l'autre, par exemple par soudage, à l'extrémité 49. Chaque lame 47,48 fait office à la fois d'élément écarteur dans une zone proche de l'extrémité commune 49 et de tige d'appui vers son extrémité libre, comme on peut le constater à l'examen de la figure 6B qui représente ledit dispositif 46 placé à l'intérieur d'une veine 50 : les parties extrêmes 51,52 des deux lames 47,48 sont appliquées sur les bords 53,54 de la veine 50 qu'elles repoussent sous l'effet du ressort des parties 55,56 proches de l'extrémité commune 49. De préférence ces parties 55,56 sont doublées par des lames de renfort.

Ce dernier mode de réalisation est très souple et son introduction dans la veine est facilitée du fait que les deux lames peuvent être aisément repliées à l'intérieur du cathéter d'introduction.

La mise en place du dispositif de l'invention est conforme à ce qui se pratique dans le domaine des filtres. Elle se fait sur une veine dénudée ou au travers d'un trocart. On introduit dans la veine, jusqu'à l'endroit du largage du dispositif, un cathéter porteur de conduites, flexibles , éventuellement avec une soudure prédéterminée, avec ou sans mandrin, avec ou sans fil de guidage . Afin d'orienter le dispositif afin que le plan général de celui-ci soit selon la direction des deux bords veineux, le cathéter peut être aplati à son extrémité distale ou encore avoir sa surface intérieure rainurée.

Une tige poussoir, disposée à l'intérieur du cathéter porteur, permet de repousser le dispositif et de larguer celui-ci hors du cathéter.

On comprend que l'extrémité distale de la tige poussoir doit venir en contact avec le dispositif pour réaliser cette expulsion. Afin de parfaire l'orientation du dispositif lors de son largage , l'extrémité distale de la tige poussoir peut avantageusement être conformée en sorte de coopérer avec la zone du dispositif qui est directement en contact avec ladite tige, en sorte qu'une rotation de la tige poussoir entraîne une rotation correspondante du dispositif. Par exemple la tige poussoir présente à son extrémité un ergot aplati, tandis que la zone en regard du dispositif comporte une fente complémentaire dudit ergot. Ainsi l'extrémité distale de la tige poussoir peut pénétrer dans le dispositif et le faire tourner, puis par simple retrait de la tige se désolidariser du dispositif.

De même pour le mode de réalisation montré à la figure 8, l'extrémité de la tige poussoir peut contribuer à effectuer, le dispositif étant en place , le réglage de l'écartement des tiges d'appui. Il suffit pour cela que la vis de réglage 37 soit pourvue d'une encoche dans laquelle peut pénétrer l'extrémité de la tige poussoir.

Bien sûr le largage du dispositif, réalisé selon le même principe que celui des filtres, peut être accompagné d'injections de médicaments sclérosants, anticoagulants ou autres. Pour faciliter ce largage, le cathéter et la tige poussoir peuvent avoir des repères visibles à leurs extrémités proximales à l'extérieur du patient, indiquant notamment l'orientation du dispositif près de leurs extrémités distales à l'intérieur dudit patient.

Lorsque le dispositif comporte des moyens d'ancrage , on utilise une double tige poussoir, ce qui évite le frottement desdits moyens d'ancrage le long de la surface intérieure du cathéter porteur.

Le dispositif selon l'invention peut être réalisé, en tout ou partie , à l'aide de matériaux permettant la diffusion de produits médicamenteux, tels qu'anti-inflammatoires, sclérosants, vasoconstricteurs ou autres. Il peut s'agir de résine ou de colle biologique.

Un tel dispositif peut également être réalisé , en tout ou partie, à l'aide d'un matériau résorbable , tel que du catgut.

L'invention n'est pas limitée aux modes de réalisation qui viennent d'être décrits à titre d'exemples non exhaustifs. Elle peut être notamment réalisée soit sous une forme monobloc soit être composée de plusieurs éléments, chacun de ces éléments pouvant être dans un matériau différent par exemple en acier inoxydable, en titane , en matériau polymérique... Les matériaux utilisés peuvent être à mémoire de formes ou encore avoir subi un traitement de surface pour augmenter les réactions de sclérose, par exemple avec du cuivre ou au contraire pour augmenter sa biocompatibilité avec par exemple des fluorocarbones héparines.

Le dispositif selon l'invention peut également comporter, au niveau de son élément écarteur , un anneau ou un crochet pour faciliter son largage ou sa récupération. De plus , il peut posséder des moyens permettant son repérage à l'intérieur du patient tels que des composants radio-opaques, des composants à forte échogénicité, des composants colorimétriques pour une bonne visualisation en endoscopie veineuse, ou encore des composants conducteurs de lumière pour un repérage par translumination cutanée. Le dispositif de l'invention apte à réaliser l'aplatissement partiel ou total d'une veine trouvera son intérêt pour le traitement des varices , des dilatations veineuses , des angiomes , des incontinences chroniques des voies veineuses profondes , de la consolidation valvulaire et de la sclérose, sans que cette liste soit exhaustive.

## Revendications

1. Dispositif intraveineux (4) pour l'aplatissement partiel ou total d'une veine du type prenant appui sur la paroi interne de la veine, par effet de ressort, caractérisé en ce qu'il comporte au moins deux tiges d'appui (1,2), qui sont reliées par au moins un élément écarteur (3) formant ressort et qui sont aptes à prendre appui respectivement sur l'un et l'autre des deux bords opposés d'une veine et à les éloigner l'un de l'autre et en ce qu'il est sensiblement plan quand il est positionné dans la veine, lesdites tiges étant sensiblement parallèles.

2. Dispositif intraveineux (5) selon la revendication 1 caractérisé en ce qu'il comporte quatre tiges d'appui (6) disposées aux extrémités d'un élément écarteur en forme de X (7).

3. Dispositif intraveineux (8) selon la revendication 1 caractérisé en ce que l'élément écarteur comporte des portions (9,10) de préférence aplaites, aptes à faire office de tiges d'appui.

4. Dispositif intraveineux (8) selon la revendication 3 caractérisé en ce qu'il a la forme d'une ellipse allongée , et en ce que les deux tiges d'appui sont constituées par les portions (9,10) en vis-a-vis sensiblement parallèles au grand axe (11) de l'ellipse.

5. Dispositif intraveineux (46) selon la revendication 3 caractérisé en ce qu'il a la forme d'un V, étant constitué de deux lames (47,48) solidarisées l'une contre l'autre, à une extrémité (49) , la portion extrême (51,52) de chaque lame (47,48) faisant office de tige d'appui.

6. Dispositif intraveineux selon l'une des revendications 1 à 5 caractérisé en ce que tout ou partie des tiges d'appui sont équipées d'éléments d'ancrage (14,19).

7. Dispositif intraveineux selon la revendication 1 caractérisé en ce que chaque tige d'appui comporte des ondulations (45).

8. Dispositif intraveineux (28) selon la revendication 1 caractérisé en ce qu'il comporte des moyens de réglage (29) de l'écartement des tiges d'appui (32,33).

9. Dispositif intraveineux (28) selon la revendication 8 caractérisé en ce que l'élément écarteur ayant la forme d'un losange, les moyens de réglage consistent en un système de vis sans fin (36) ou de crémaillère, placé selon l'axe longitudinal du losange.

10. Dispositif intraveineux (23) selon la revendication 1 caractérisé en ce qu'il a la forme d'un H, avec une ou deux barres transversales (26,27), et en ce que la ou les barres dudit H constituant l'élément écarteur sont courbes et ne sont pas dans le plan des tiges d'appui (24,25).

11. Dispositif intraveineux selon la revendication 10 caractérisé en ce que, étant destinée à être placée au niveau d'une valvule , la ou les barres transversales ont une courbure concave apte à épouser la forme d'un ou des deux bourrelets valvulaires.

12. Dispositif intraveineux selon la revendication 1 caractérisé en ce que l'une au moins des tiges ou l'élément écarteur est dans un matériau apte à contenir et diffuser des produits médicamenteux.

13. Dispositif intraveineux selon la revendication 1 caractérisé en ce qu'il est au moins partiellement résorbable.

14. Dispositif intraveineux (38) selon la revendication 1 caractérisé en ce qu'il est composé de deux ensembles distincts, chacun étant constitué d'au moins deux tiges et d'un élément écarteur , en ce que lesdits ensembles sont reliés par leurs éléments écarteurs à l'aide d'un lien résorbable (43) , en ce que les quatre tiges sont munies de moyens d'ancrage (44) et en ce que le lien résorbable (43) est imprégné d'un produit apte à réaliser la section ou la sclérose chimique de la paroi veineuse.

## Claims

1. Intravenous device (4) for partially or totally flattening a vein, of the type bearing against the inner wall of the vein, by a springlike effect, being characterized in that it comprises least two presser rods (1, 2) that are interconnected by at least one spring-forming spreader element (3) and that are respectively suitable for bearing against both of the opposite borders of a vein, and for urging them away from each other and in that it is substantially plane when it is in position in a vein, said rods being substantially parallel.

2. Intravenous device (5) according to claim 1, characterized in that it comprises four presser rods (6) disposed at the ends of an X-shaped spreader element (7).

3. Intravenous device (8) according to claim 1, characterized in that the spreader element includes preferably flattened portions (9, 10) suitable for acting as presser rods.

4. Intravenous device (8) according to claim 3, characterized in that it is in the form of an elongated ellipse, and in that the two presser rods are constituted by the facing portions (9,10) that are substantially parallel to the major axis (11) of the ellipse.

5. Intravenous device (46) according to claim 3, characterized in that it is V-shaped, being constituted by two blades (47, 48) secured one against the other at one end (49), the end portion (51, 52) of each blade (47, 48) acting as a presser rod.

6. Intravenous device according to one of claims 1 to 5, characterized in that all or a portion of the presser rods are fitted with anchor elements (14, 19).

7. Intravenous device according to claim 1, characterized in that each presser rod comprises corrugations (45).

8. Intravenous device (28) according to claim 1, characterized in that it comprises adjustment means (29) for adjusting the spacing between the presser rods (32, 33).

9. Intravenous device (28) according to claim 8, characterized in that the spreader element is in the form of a lozenge, the adjustment means consisting in a worm screw (36) or rack system extending along the longitudinal axis of the lozenge.

10. Intravenous device (23) according to claim 1, characterized in that it is H-shaped, having one or two crossbars (26, 27), and in that the bar or bars of said H-shape constituting the spreader element are curved and do not lie in the plane of the presser rods (24, 25).

11. Intravenous device according to claim 10, characterized in that for placing at a valve, the crossbar or crossbars are of concave curvature suitable for fitting closely to the shape of one or both valvular thickenings.

12. Intravenous device according to claim 1, characterized in that at least one of the rods or the spreader element is made of a material suitable for containing and diffusing medicinal substances.

13. Intravenous device according to claim 1, characterized in that it is at least partly resorbable.

14. Intravenous device (38) according to claim 1, characterized in that it is made up of two distinct assemblies, each of which is constituted by at least two rods and a spreader element, in that said assemblies have their spreader elements connected together by means of a resorbable link (43), in that the four rods are provided with respective anchoring means (44), and in that the resorbable link (43) is impregnated with a substance suitable for chemically sclerosing or sectioning the wall of a vein.

## Patentansprüche

1. Intravenöse Vorrichtung (4) zum teilweisen oder vollständigen Abflachen einer Vene des Typs, der sich unter Federeinwirkung an die Innenwand der Vene anlegt, dadurch gekennzeichnet, daß sie mindestens zwei Anlagestifte (1, 2) aufweist, die durch mindestens einen Abstandhalter (3) verbunden sind, der eine Feder bildet, und die geeignet sind, an dem einen bzw. dem anderen der beiden gegenüberliegenden Ränder einer Vene in Anlage zu kommen und diese auseinanderzudrücken, und daß sie im wesentlichen flach ist, wenn sie in der Vene angeordnet wird, wobei die genannten Stifte im wesentlichen parallel zueinander liegen.

2. Intravenöse Vorrichtung (5) nach Anspruch 1, dadurch gekennzeichnet, daß sie mindestens zwei Anlagestifte (6) aufweist, die an den Enden eines X-förmigen Abstandhalters (7) angeordnet sind.

3. Intravenöse Vorrichtung (8) nach Anspruch 1, dadurch gekennzeichnet, daß der Abstandhalter Abschnitte (9, 10) aufweist, die vorzugsweise abgeflacht sind und geeignet sind, als Anlagestifte zu dienen.

4. Intravenöse Vorrichtung (8) nach Anspruch 3, dadurch gekennzeichnet, daß sie die Form einer länglichen Ellipse hat und daß die beiden Anlagestifte aus den einander gegenüberliegenden, im wesentlichen parallel zur langen Achse (11) der Ellipse verlaufenden Abschnitten (9, 10) bestehen.

5. Intravenöse Vorrichtung (46) nach Anspruch 3, dadurch gekennzeichnet, daß sie die Form eines V hat und aus zwei Plättchen (47, 48) besteht, die an einem Ende (49) fest miteinander verbunden sind, wobei der äußere Abschnitt (51, 52) jedes Plättchens (47, 48) als Anlagestift dient.

6. Intravenöse Vorrichtung nach einem der Ansprüche 1 bis 5, dadurch gekennzeichnet, daß alle oder ein Teil der Anlagestifte mit Verankerungselementen (14, 19) versehen sind.

7. Intravenöse Vorrichtung nach Anspruch 1, dadurch gekennzeichnet, daß jeder Anlagestift Wellen (45) aufweist.

8. Intravenöse Vorrichtung (28) nach Anspruch 1, dadurch gekennzeichnet, daß sie Einstellmittel (29) für den Abstand der Anlagestifte (32, 33) voneinander aufweist.

9. Intravenöse Vorrichtung (28) nach Anspruch 8, dadurch gekennzeichnet, daß bei einem rautenförmigen Abstandhalter die Einstellmittel aus einem Schneckensystem (36) oder Zahnstangensystem bestehen, das auf der Längsachse der Raute angeordnet ist.

10. Intravenöse Vorrichtung (23) nach Anspruch 1, dadurch gekennzeichnet, daß sie die Form eines H mit einen oder zwei Querbalken (26, 27) hat und daß der oder die Balken des genannten H, die den Abstandhalter bilden, gebogen sind und sich nicht in der Ebene der Anlagestifte (24, 25) befinden.

11. Intravenöse Vorrichtung nach Anspruch 10, dadurch gekennzeichnet, daß der oder die Querbalken, die im Bereich einer Klappe angebracht werden sollen, eine konkave Krümmung aufweisen, die geeignet ist, sich an die Form einer oder beider Klappenwülste anzulegen.

12. Intravenöse Vorrichtung nach Anspruch 1, dadurch gekennzeichnet, daß mindestens einer der Stifte oder der Abstandhalter aus einem Material besteht, das geeignet ist, medikamentöse Mittel zu enthalten und abzugeben.

13. Intravenöse Vorrichtung nach Anspruch 1, dadurch gekennzeichnet, daß sie zumindest teilweise resorbiert werden kann.

14. Intravenöse Vorrichtung (38) nach Anspruch 1, dadurch gekennzeichnet, daß sie aus zwei verschiedenen Einheiten besteht, die jeweils aus mindestens zwei Stiften und einem Abstandhalter bestehen, daß die genannten Einheiten an ihren Abstandhaltern mittels einer resorbierbaren Verbindung (43) miteinander verbunden sind, daß die vier Stifte mit Verankerungsmitteln (44) versehen sind und daß die resorbierbare Verbindung (43) mit einem Produkt getränkt ist, das geeignet ist, den Schnitt oder die chemische Verhärtung der Venenwand durchzuführen.
